(19) Europäisches Patentamt
European Patent Office
Office européen des brevets

(11) Veröffentlichungsnummer: **0 008 343**
**B1**

# (12) EUROPÄISCHE PATENTSCHRIFT

(45) Veröffentlichungstag der Patentschrift: **06.04.83**

(51) Int. Cl.³: **C 07 D 501/22,** A 61 K 31/545

(21) Anmeldenummer: **79102273.4**

(22) Anmeldetag: **04.07.79**

## (54) Cephalosporinderivate, Verfahren zu ihrer Herstellung und diese enthaltende pharmazeutische Präparate.

(30) Priorität: **07.07.78 CH 7422/78**
**21.12.78 CH 13016/78**

(43) Veröffentlichungstag der Anmeldung:
**05.03.80 Patentblatt 80/5**

(45) Bekanntmachung des Hinweises auf die Patenterteilung:
**06.04.83 Patentblatt 83/14**

(84) Benannte Vertragsstaaten:
**LU**

(56) Entgegenhaltungen:
**EP-A-0 001 125**
**DE-A-2 710 902**
**DE-A-2 728 766**

(73) Patentinhaber: **CIBA-GEIGY AG, Patentabteilung Postfach, CH-4002 Basel (CH)**

(72) Erfinder: **Scartazzini, Riccardo, Dr., Conrad Ferdinand Meyer-Strasse 38, CH-4059 Basel (CH)**
Erfinder: **Wiederkehr, René, Dr., Grenzweg 9, CH-4148 Pfeffingen (CH)**

(74) Vertreter: **Zumstein, Fritz sen., Dr. et al, Bräuhausstrasse 4, D-8000 München 2 (DE)**

**EP 0 008 343 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Cephalosporinderivate, Verfahren zu ihrer Herstellung und diese enthaltende pharmazeutische Präparate

Die Erfindung betrifft neue 7β-Aminothiazolyl-acetamido-3-cephem-4-carbonsäure-verbindungen, die in 2- und in 3-Stellung des Cephemringes unsubstituiert sind, Verfahren zu ihrer Herstellung, pharmazeutische Präparate die solche Verbindungen enthalten und diese Verbindungen zur Verwendung als Antibiotika.

Die Erfindung betrifft insbesondere 7β-Amino-thiazolyl-acetamido-3-cephem-4-carbonsäureverbindungen der Formel

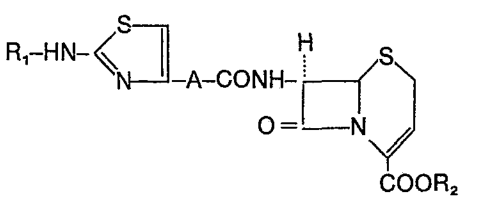

(I),

worin $R_1$ Wasserstoff oder eine Aminoschutzgruppe, A durch Methoxyimino substituiertes Methylen und $R_2$ Pivaloyloxymethyl darstellt, und Salze von solchen Verbindungen mit salzbildenden Gruppen.

In der vorliegenden Beschreibung der Erfindung bedeutet der Ausdruck «Nieder» in Gruppen wie Niederalkyl, Niederalkoxy, Niederalkanoyl und dergleichen, dass die entsprechenden Gruppen, sofern nicht ausdrücklich anders definiert, bis zu 7, bevorzugt bis zu 4 C-Atome enthalten.

Die in Verbindungen der Formel I vorhandene Aminogruppe ist gegebenenfalls durch eine Schutzgruppe geschützt, die in der Penicillin-, Cephalosporin- und Peptidchemie verwendet wird.

Eine solche Schutzgruppe ist leicht, das heisst ohne dass unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise beschrieben in «Protective Groups in Organic Chemistry», Plenum Press, London, New York, 1973, ferner in «The Peptides», Vol. I, Schröder and Lübke, Academic Press, London, New York, 1965.

Eine Aminoschutzgruppe $R_1$ ist in erster Linie eine Acylgruppe Ac, eine Mono-, Di- oder Triarylmethylgruppe oder eine Silyl- oder Stannylgruppe.

Eine Acylgruppe Ac ist der Acylrest einer organischen Carbonsäure mit bis zu 18 C-Atomen, insbesondere einer gegebenenfalls, z.B. durch Halogen oder Aryl, substituierten aliphatischen Carbonsäure oder gegebenenfalls, z.B. durch Halogen, Niederalkoxy oder Nitro, substituierten aromatischen Carbonsäure, oder eines Kohlensäurehalbesters. Solche Acylgruppen sind beispielsweise Niederalkanoyl, wie Formyl, Acetyl, Propionyl, Halogenniederalkanoyl, wie 2-Halogenacetyl, insbesondere 2-Chlor-, 2-Brom-, 2-Jod, 2,2-Dichlor- oder 2,2,2-Trichloracetyl, Phenylacetyl, Phenoxyacetyl, Thienylacetyl, Benzoyl, 4-Chlor-, 4-Methoxy- oder 4-Nitrobenzoyl, Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro-, mono- oder polysubstituierte Phenylreste darstellt, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, oder 2-Halogen-niederalkoxycarbonyl, z.B. 2,2,2-Trichloräthoxycarbonyl, 2-Chloräthoxycarbonyl, 2-Bromäthoxycarbonyl oder 2-Iodäthoxycarbonyl, oder Acylmethoxycarbonyl, insbesondere Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl.

In einer Mono-, Di- oder Triarylmethylgruppe $R_1$ sind die Arylreste insbesondere gegebenenfalls substituierte Phenylreste. Solche Gruppen sind beispielsweise Benzyl, Diphenylmethyl oder Trityl.

Eine Silyl- oder Stannylgruppe $R_1$ ist in erster Linie eine organische Silyl- bzw. Stannylgruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxyniederalkyl-halogen-silyl, z.B. Methoxy-methyl-chlor-silyl, oder Diniederalkyl-halogen-silyl, z.B. Dimethyl-chlor-silyl, oder entsprechend substituiertes Stannyl, z.B. Tri-n-butylstannyl.

Bevorzugte Aminoschutzgruppen $R_1$ sind die Acylreste der 2-Halogenessigsäure, insbesondere 2-Chloracetyl, sowie von Kohlensäurehalbestern, insbesondere tert.-Niederalkoxycarbonyl, z.B. tert.-Butoxycarbonyl, gegebenenfalls, z.B. wie angegeben substituiertes Benzyloxycarbonyl oder Diphenylmethoxycarbonyl, oder 2-Halogen-niederalkoxycarbonyl, wie 2,2,2-Trichloräthoxycarbonyl, sowie die Tritylgruppe.

Die gleichen Aminoschutzgruppen können in einer im Rest A befindlichen geschützten Aminogruppe vorhanden sein.

Eine Aminogruppe kann auch in protonierter Form geschützt werden; als Anionen kommen in erster Linie diejenigen von starken anorganischen Säuren, wie von Halogenwasserstoffsäuren, z.B. das Chlor- oder Bromanion, oder von Sulfonsäuren, wie p-Toluolsulfonsäure, in Frage.

Verbindungen der Formel I mit einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin bilden.

Die Methoxyiminomethylengruppe A liegt bevorzugt in der syn-Form (Z-Form) vor.

Die Verbindungen der Formel I und ihre pharmazeutisch verwendbaren, nichttoxischen Salze sind wertvolle, antibiotisch wirksame Substanzen, die insbesondere als antibakterielle Antibiotika verwendet werden können. Beispielsweise sind sie in vitro gegen grampositive und gramnegative Kokken, wie Staphylococcus aureus, Streptococcus pyogenes, Streptococcus faecalis, Streptococcus pneumoniae, Neisseria gonorrhoeae und Neisseria meningitidis in Minimalkonzentrationen von etwa 0,005 bis 1µg/ml und gegen gram-negative Bakterien, wie Escherichia coli, Klebsiella pneumoniae, Serratia marcescens, Enterobacter sp., Proteus sp. indol+ und indol−, Pseudomonas aeruginosa und Haemophilus influenzae, inklusive gegen β-Laktamase produzierende Stämme, in Minimalkonzentrationen von <0,01 bis 8 µg/ml wirksam. In vivo, bei subcutaner Applikation an der Maus, sind sie beispielsweise gegen Enterobakterien, sowie gegen übrige gramnegative und grampositive Erreger in Minimaldosen von $ED_{50}$ <0,1 mg bis 100 mg/kg wirksam. Die neuen Verbindungen können auch deshalb entsprechend, z.B. in Form von antibiotisch wirksamen Präparaten, zur Behandlung von durch grampositive oder insbesondere gramnegative Infektionserreger, insbesondere durch von Enterobakterien, wie Escherichia coli, Klebsiella und Proteus (indol + indol −) verursachten Infektionen Verwendung finden.

Verbindungen der Formel (I), worin die funktionellen Gruppen geschützt sind, werden als Ausgangsmaterialien zur Herstellung von antibiotisch wirksamen Verbindungen der Formel I verwendet.

Die vorliegende Erfindung betrifft in erster Linie diejenigen Verbindungen der Formel I, worin $R_1$ insbesondere Wasserstoff, sowie 2-Halogenniederalkanoyl, wie 2-Chlorniederalkanoyl, bedeutet, A Methoxyiminomethylen darstellt, und $R_2$ unter physiologischen Bedingunggen abspaltbares Pivaloyloxymethyl bedeutet, und ihre pharmazeutisch annehmbaren Salze, sowie die entsprechenden Verbindungen mit geschützten funktionellen Gruppen.

Die Erfindung betrifft insbesondere die in den Beispielen beschriebenen Verbindungen der Formel I und deren pharmazeutisch annehmbaren Salze.

Die Verbindungen der vorliegenden Erfindung werden nach an sich bekannten Verfahren hergestellt.

So werden Verbindungen der Formel I hergestellt, indem man zur Herstellung einer Verbindung der Formel I, worin $R_1$ Wasserstoff ist, und worin A und $R_2$ die unter Formel I gegebenen Bedeutungen haben, aus einer Verbindung der Formel I, worin $R_1$ eine Aminoschutzgruppe bedeutet, und A und $R_2$ die unter Formel I gegebenen Bedeutungen haben, die Aminoschutzgruppe $R_1$ abspaltet und durch Wasserstoff ersetzt, oder zur Herstellung einer Verbindung der Formel I, worin $R_2$ Pivaloyloxymethyl darstellt, und $R_1$ und A die unter Formel I genannten Bedeutungen haben, in einer Verbindung der Formel I, worin $R_2$ Wasserstoff ist, und worin $R_1$ und A die unter Formel I gegebenen Bedeutungen haben, die freie Carboxylgruppe in 4-Stellung des Cephemringes, oder ein reaktionsfähiges funktionelles Derivat davon, durch Behandlung mit einem den Pivaloyloxymethylrest einführenden Veresterungsmittel in eine veresterte Carboxylgruppe überführt.

Die Abspaltung einer Aminoschutzgruppe $R_1$ aus einer Verbindung der Formel I, erfolgt, gegebenenfalls selektiv, auf an sich bekannte und, je nach Art der Schutzgruppe, verschiedenartige Weise, z.B. mittels Solvolyse oder Reduktion. Eine 2-Halogen-Niederalkoxycarbonylgruppe (gegebenenfalls nach Umwandlung einer 2-Brom-niederalkoxycarbonylgruppe in eine 2-Jod-niederalkoxycarbonylgruppe), eine Acylmethoxycarbonylgruppe oder eine 4-Nitrobenzyloxycarbonylgruppe $R_1$ kann z.B. durch Behandeln mit einem geeigneten chemischen Reduktionsmittel, wie Zink in Gegenwart von wässriger Essigsäure, eine Aroylmethoxycarbonylgruppe auch durch Behandeln mit einem nucleophilen, vorzugsweise salzbildenden Reagens, wie Natriumthiophenolat, und eine 4-Nitro-benzyloxycarbonylgruppe auch durch Behandeln mit einem Alkalimetall-, z.B. Natriumdithionit, eine Diphenylmethoxycarbonyl-, tert.-Niederalkoxycarbonyl- oder Polycycloalkoxycarbonylgruppe durch Behandeln z.B. mit Ameisen- oder Trifluoressigsäure, eine gegebenenfalls substituierte Benzyloxycarbonylgruppe z.B. mittels Hydrogenolyse durch Behandeln mit Wasserstoff in Gegenwart eines Hydrierkatalysators, wie eines Palladiumkatalysators, eine Trialkylmethylgruppe z.B. durch Behandeln mit wässriger Mineralsäure, und eine organische Silyl- oder Stannylgruppe $R_1$ z.B. mittels Hydrolyse oder Alkoholyse abgespalten und durch Wasserstoff ersetzt werden. Eine 2-Halogenacetyl-, wie 2-Chloracetylgruppe $R_1$ kann durch Behandeln mit Thioharnstoff in Gegenwart einer Base, oder mit einem Thiolatsalz, wie einem Alkalimetallthiolat des Thioharnstoffs und anschliessende Solvolyse, wie Alkoholyse oder Hydrolyse, des entstandenen Kondensationsproduktes abgespalten werden.

Diese Solvolyse- oder Reduktionsreaktionen erfolgen gegebenenfalls in Gegenwart eines an der Reaktion beteiligten oder auch eines inerten Lösungsmittels unter Kühlen oder Erwärmen, z.B. bei Temperaturen von etwa −20° bis etwa 100°.

Die Veresterung einer freien Carboxylgruppe, oder eines reaktionsfähigen funktionellen Derivates davon, in 4-Stellung des Cephemringes in einer Verbindung der Formel I erfolgt, gegebenenfalls unter Schutz anderer funktioneller Gruppen, auf an sich bekannte Weise.

So erhält man Ester z.B. durch Behandeln einer freien Carboxylgruppe –COOH oder eines Salzes der Säure mit einem reaktionsfähigen Ester eines Alkohols der Formel $R_2$–OH z.B. einem Halogen-, wie Chlor- oder Jodmethylpivalat.

Die Veresterungsreaktion kann in An- oder Abwesenheit eines inerten Lösungsmittels, wie eines gegebenenfalls halogenierten Kohlenwasserstoffes, z.B. Methylenchlorid, Benzol, Chlorbenzol, eines Amides, z.B. Dimethylformamid, eines Sulfoxids, z.B. Dimethylsulfoxid, eines Nitriles z.B. Acetonitril, oder dergleichen, oder gegebenenfalls auch in einem Überschuss des Alkoholes $R_2$–OH oder des Derivates davon unter Kühlen oder Erwärmen, z.B. je nach Methode zwischen etwa $-50°$ und 100°, durchgeführt werden.

Salze von Verbindungen der Formel (I) können in an sich bekannter Weise hergestellt werden.

Die Verfahren umfassen auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder in situ, gegebenenfalls unter den Reaktionsbedingungen, gebildet werden.

Die Ausgangsverbindungen der Formel I, worin $R_1$ eine Aminoschutzgruppe darstellt, A und $R_2$ die unter Formel I gegebenen Bedeutungen haben, und Salze von solchen Verbindungen können beispielsweise hergestellt werden, indem man die 7β-Aminogruppe in einer Verbindung der Formel

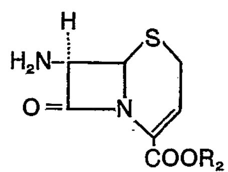

(II),

worin die Aminogruppe gegebenenfalls durch eine die Acylierung erlaubende Gruppe substituiert ist und worin $R_2$ die unter Formel I gegebenen Bedeutungen hat, durch Behandeln mit einem den Acylrest einer Carbonsäure der Formel

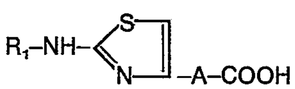

(III)

einführenden Acylierungsmittel, worin $R_1$ eine Aminoschutzgruppe ist und A die unter Formel I angegebenen Bedeutungen hat und worin gegebenenfalls vorhandene funktionelle Gruppen in geschützter Form vorliegen, acyliert, oder zur Herstellung von Ausgangsverbindungen der Formel I, worin $R_2$ Wasserstoff ist, und $R_1$ und A die unter Formel I genannten Bedeutungen haben, die Aminogruppe in einer Verbindung der Formel II, worin die Aminogruppe gegebenenfalls durch eine die Acylierung erlaubende Gruppe substituiert ist und $R_2$ Wasserstoff oder einen die Carboxylgruppe veresternden Rest mit Ausnahme von Pivaloyloxymethyl darstellt, durch Behandeln mit einem den Acylrest einer Carbonsäure der Formel III einführenden Acylierungsmittel, worin A und $R_1$ die unter Formel I angegebenen Bedeutungen haben und worin gegebenenfalls vorhandene funktionelle Gruppen in geschützter Form vorliegen, acyliert und in einer erhaltenen Verbindung der Formel I, worin $R_2$ einen die Carboxylgruppe veresternden Rest darstellt, und $R_1$ und A die unter Formel I genannten Bedeutungen haben, diesen Rest $R_2$ abspaltet und durch Wasserstoff oder ein Kation ersetzt.

Der die Carboxylgruppe veresternde Rest $R_2$ ist bevorzugt unter schonenden Bedingungen, inklusive unter physiologischen Bedingungen, leicht abspaltbar. Solche Reste $R_2$ sind beispielsweise Niederalkyl, insbesondere tert.-Niederalkyl, z.B. tert.-Butyl, Polycycloalkyl, z.B. Adamantyl, Arylmethyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls, z.B. wie oben erwähnt substituiertes Benzyl, z.B. 4-Nitro-benzyl, oder 4-Methoxybenzyl, oder z.B. wie oben erwähnt substituiertes Diphenylmethyl, z.B. Benzhydryl oder Di(4-methoxyphenyl)-methyl, oder 2-Halogenniederalkyl, z.B. 2,2,2-Trichloräthyl, insbesondere Aroylmethyl, worin die Aroylgruppe vorzugsweise gegebenenfalls, z.B. durch Halogen, wie Brom, substituiertes Benzoyl darstellt, z.B. Phenacyl, oder Polyhalogenaryl, wie Pentachlorphenyl. $R_2$ ist ferner eine Silyl-, insbesondere eine organische Silylgruppe oder eine entsprechende Stannylgruppe. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.-butyl-silyl, Niederalkoxyniederalkyl-halogen-silyl, z.B. Methoxy-methyl-chlor-silyl, oder Diniederalkyl-halogen-silyl, z.B. Dimethylchlor-silyl, oder entsprechend substituierte Stannylverbindungen, z.B. Tri-n-butylstannyl.

Acylierung: Gegebenenfalls vorhandene, die Aminogruppe substituierende und deren Acylierung erlaubende Reste in einem Ausgangsmaterial der Formel II sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen. Bei der Silylierung oder Stannylierung einer Carboxylgruppe in einem Ausgangsmaterial der Formel II, kann, bei Verwendung eines Überschusses des Silylierungs- oder Stannylierungsmittels, die Aminogruppe ebenfalls silyliert oder stannyliert werden.

Die genannten Ylidengruppen sind in erster Linie Arylmethylengruppen, worin Aryl insbesondere für einen carbocyclischen, in erster Linie monocyclischen Arylrest, z.B. für gegebenenfalls, wie durch Nitro oder Hydroxy, substituiertes Phenyl steht; solche Arylmethylgruppen sind z.B. Benzyliden, 2-Hydroxybenzyliden oder 4-Nitrobenzyliden, ferner gegebenenfalls, z.B. durch Carboxy substituiertes Oxacycloalkyliden, z.B. 3-Carboxy-2-oxacyclohexyliden.

Den Acylrest einer Carbonsäure der Formel (III) einführende Acylierungsmittel sind beispielsweise die Carbonsäure selbst oder reaktionsfähige funktionelle Derivate davon.

Falls eine freie Säure der Formel III, worin alle funktionellen Gruppen ausser der reagierenden Carboxylgruppe geschützt sind, als Acylierungsmittel eingesetzt wird, verwendet man üblicherweise geeignete Kondensationsmittel, wie Carbodiimide, beispielsweise N,N'-Diäthyl-, N,N'-Diproypl-, N,N'-Diisopropyl-, N,N'-Dicyclohexyl- oder N-Äthyl-N'-3-dimethylaminopropyl-carbodiimid, geeignete Carbonylverbindungen, beispielsweise Carbonyldiimidazol, oder Isoxazoliniumsalze, beispielsweise N-Äthyl-5-phenyl-isoxazolinium-3'-sulfonat und N-tert.-Butyl-5-methylisoxazoliniumperchlorat, oder eine Acylaminoverbindung, z.B. 2-Äthoxy-1-äthoxycarbonyl-1,2-dihydro-chinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. Amid-bildendes, bzw. Esterbildendes, funktionelles Derivat einer Säure der Formel III, worin alle funktionellen Gruppen ausser der reagierenden Säuregruppe geschützt sind bzw. sein können, ist in erster Linie ein Anhydrid einer solchen Säure, inklusive und vorzugsweise ein gemischtes Anhydrid, aber auch ein inneres Anhydrid, d.h. ein entsprechendes Keten. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Äthyl- oder Isobutylhalbester der Kohlensäure, oder mit organischen, insbesondere aliphatischen oder aromatischen Sulfonsäuren, z.B. p-Toluolsulfonsäure. Von der Säure III, wenn A Hydroxymethylen ist, kann auch ein gemischtes inneres Anhydrid mit dem Kohlensäurehalbester der α-Hydroxygruppe verwendet werden.

Weitere reaktionsfähige Säurederivate einer Säure der Formel III, sind aktivierte Ester, wie Ester mit vinylogen Alkoholen (d.h. Enolen), wie vinylogen Niederalkenolen, oder Arylester, wie 4-Nitrophenyl- oder 2,4-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Äthyl-diisopropylamin, oder N,N-Diniederalkylanilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Äthylenoxid oder Propylenoxid, durchgeführt.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig, bei erniedrigter oder erhöhter Temperatur, etwa bei −40° bis etwa 100°, bevorzugt bei −10° bis +40°, und/oder in einer Inertgas-, z.B. Stickstoffatmosphäre.

In einer acylierenden Säure der Formel III oder in einem Säurederivat davon kann eine geschützte Aminogruppe auch in ionischer Form vorliegen, d.h. das Ausgangsmaterial der Formel III kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, in situ gebildet werden. So erhält man z.B. ein gemischtes Anhydrid durch Behandeln einer Säure der Formel III mit entsprechend geschützten funktionellen Gruppen, oder eines geeigneten Salzes davon, wie eines Ammoniumsalzes, z.B. mit einem organischen Amin, wie 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, oder mit einem Halbester eines Kohlensäurehalbhalogenids, z.B. Chlorameisensäureäthylester oder -isobutylester, und verwendet das so erhältliche gemischte Anhydrid ohne Isolierung.

Die Überführung einer veresterten Carboxylgruppe $-COOR_2$, unter Abspaltung des veresternden Restes $R_2$, in eine freie oder eine in Salzform vorliegende Carboxylgruppe, d.h. worin $R_2$ Was-

serstoff oder ein Kation ist, in einer Verbindung der Formel I, erfolgt auf an sich bekannte Weise, insbesondere durch Solvolyse, wie Hydrolyse, Alkoholyse oder Acidolyse, oder durch Reduktion, wie Hydrogenolyse oder chemische Reduktion.

So kann man z.B. eine tert.-Niederalkoxycarbonyl-, Polycycloalkoxycarbonyl- oder Diphenylmethoxycarbonylgruppe durch Behandeln mit einem geeigneten sauren Mittel, wie Ameisensäure oder Trifluoressigsäure, gegebenenfalls unter Zugabe einer nucleophilen Verbindung, wie Phenol oder Anisol, in die freie Carboxylgruppe überführen. Eine gegebenenfalls substituierte Benzyloxycarbonylgruppe kann z.B. mittels Hydrogenolyse durch Behandeln mit Wasserstoff in Gegenwart eines Hydrierkatalysators, wie eines Palladiumkatalysators freigesetzt werden. Ferner kann man bestimmt substituierte Benzyloxycarbonylgruppen, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-IIsalz, z.B. Chrom-II-chlorid, üblicherweise in Gegenwart eines Wasserstoffabgebenden Mittels, das zusammen mit dem Metall nascierenden Wasserstoff zu erzeugen vermag, wie einer Säure, in erster Linie Essig-, sowie Ameisensäure, oder eines Alkohols, wobei man vorzugsweise Wasser zugibt, in die freie Carboxylgruppe überführen. Durch Behandeln mit einem reduzierenden Metall oder Metallsalz, wie oben beschrieben, kann man auch eine 2-Halogen-niederalkoxycarbonylgruppe (gegebenenfalls nach Umwandlung einer 2-Brom-niederalkoxycarbonylgruppe in eine 2-Iodniederalkoxycarbonylgruppe) oder eine Acylmethoxycarbonylgruppe in die freie Carboxylgruppe umwandeln, wobei eine Aroylmethoxycarbonylgruppe ebenfalls durch Behandeln mit einem nucleophilen, vorzugsweise salzbildenden Reagens, wie Natriumthiophenolat oder Natriumjodid gespalten werden kann. Eine Polyhalogenaryloxycarbonylgruppe, wie die Pentachlorphenyloxycarbonylgruppe wird unter milden basischen Bedingungen, wie durch verdünnte Natronlauge oder organische Basen in Gegenwart von Wasser, zur freien Carboxylgruppe verseift.

Eine z.B. durch Silylierung oder Stannylierung geschützte Carboxylgruppe kann in üblicher Weise, z.B. durch Behandeln mit Wasser oder einem Alkohol, freigesetzt werden.

Bei der basischen Hydrolyse oder Alkoholyse kann das Salz der Carbonsäure anfallen.

Die Reaktion erfolgt in dem Solvolysierungsmittel allein, falls es sich um ein Lösungsmittel handelt, oder gegebenenfalls in einem inerten Lösungsmittel, oder in einem Gemisch davon, je nach Methode bei erniedrigter oder erhöhter Temperatur; beispielsweise zwischen etwa −50° und 100°.

Die Ausgangsverbindungen der Formeln II und III sind bekannt oder können analog bekannten Verfahren hergestellt werden.

So sind Ausgangsverbindungen der Formel II aus der DT-OS 2 151 567 bekannt.

Ausgangsverbindungen der Formel III sind z.B. aus BE-PS 852 971, BE-PS 853 545, DE-OS 2 556 736 und DE-OS 2 638 028 bekannt.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur enteralen oder parenteralen Verabreichung eignen. Zur enteralen Verabreichung verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen oder Adsorptionsmittel, Farbstoffe, Geschmacksstoffe und Süssmittel. Suppositorien sind in erster Linie Fettemulsionen oder -suspensionen.

Vorzugsweise verwendet man die pharmakologisch wirksamen Verbindungen der vorliegenden Erfindung in Form von injizierbaren, z.B. intravenös oder subcutan, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Mischungs-, Lösungs-, oder Lyophilisierungsverfahren, hergestellt und enthalten von etwa 0,1% bis 100%, insbesondere von etwa 1% bis etwa 50%, Lyophilisate bis zu 100% des Aktivstoffes. Je nach Art der Infektion, Zustand des infizierten Organismus verwendet man tägliche Dosen von etwa 0,5 g 0,5 bis etwa 5 g s.c. zur Behandlung von Warmblütern von etwa 70 kg Gewicht.

Die folgenden Beispiele dienen zur Illustration der Erfindung; Temperaturen werden in Celsiusgraden angegeben.

Rf-Angaben für Dünnschichtchromatographie:

DS: Auf Silicagel-Fertigplatten SL 254 der Fa. Antec, Birsfelden.

Die Zusammensetzung der Laufmittel ist wie folgt:

52 A: n-Butanol/Eisessig/Wasser 67:10:23

67: n-Butanol/Äthanol/Wasser 40:10:50 (obere Phase)

101: n-Butanol/Pyridin/Eisessig/Wasser 38:24:8:30

101 A: n-Butanol/Pyridin/Eisessig/Wasser 42:24:4:30

In den Aminothiazol-2-methoxyiminoessigsäureverbindungen hat die 2-Methoxyiminogruppe die syn-Konfiguration.

Beispiel 1

a) Eine Lösung von 1,14 g 2-(2-Chloracetamido-4-thiazolyl)-2-methoxyiminoessigsäure in 15 ml Tetrahydrofuran wird bei −10° mit 0,85 g N,N'-Dicyclohexylcarbodiimid und 0,56 g 1-Hydroxybenztriazol versetzt und bei ca. −5° bis −10° unter Stickstoff 1 ½ Stunden gerührt. Nach Zugabe einer Lösung von 1,0 g 7β-Amino-3-cephem-4-carbonsäure-diphenylmethylester in 15 ml Tetrahydrofuran wird zunächst 1 Stunde bei der gleichen Temperatur und dann 4 Stunden bei Raumtemperatur weitergerührt. Das Reaktionsgemisch wird filtriert, und das Filtrat im Vakuum eingedampft. Der Eindampf-Rückstand wird in Äthylacetat aufgenommen, mit gesättigter wässriger Natriumchloridlösung gewaschen, über Natriumsulfat getrocknet, filtriert und im Vakuum eingeengt. Dieser Rückstand wird in Äthylacetat aufgenommen und nach Abfiltration des unlöslichen Materials wiederum eingedampft. Durch Chromatographie an Silikagel mit Toluol und steigenden Anteilen Äthylacetat (bis zu 30%) wird daraus der 7β-[2-(2-Chloracetamido-4-thiazolyl)-2-methoxyiminoacetamido]-3-cephem-4-carbonsäure-diphenylmethyl-ester gewonnen.

DS: Rf ~ 0,57 (Äthylacetat); IR-Spektrum ($CH_2Cl_2$): Absorptionsbanden bei 3350, 3200, 1782, 1770sh, 1724, 1705, 1684, 1540 $cm^{-1}$.

b) Eine Mischung von 1,25 g 7β-[2-(2-Chloracetamido-4-thiazolyl)-2-methoxyiminoacetamido]-3-cephem-4-carbonsäure-diphenylmethylester, 12 ml Methylenchlorid, 0,68 ml Anisol und 3,4 ml Trifluoressigsäure wird 30 Minuten unter Eiskühlung gerührt. Nach Zutropfen von 100 ml Diäthyläther wird 45 Minuten bei der gleichen Temperatur weiter gerührt. Die ausgefallene 7β-[2-(2-Chloracetamido-4-thiazolyl)-2-methoxyiminoacetamido]-3-cephem-4-carbonsäure wird abfiltriert, mit Diäthyläther gewaschen und getrocknet.

DS: Rf 0,37 (n-Butanol:Eisessig:Wasser 67:10:23).:

c) Eine Lösung von 700 mg 7β-[2-(2-Chloracetamido-4-thiazolyl)-2-methoxyiminoacetamido]-3-cephem-4-carbonsäure in 17 ml N,n'-Dimethylacetamid wird mit 260 mg Thioharnstoff versetzt und 19 Stunden bei Raumtemperatur gerührt. Der durch Zugabe von 200 ml Diäthyläther und Dekantieren des Lösungsmittels erhaltene Niederschlag wird mit 100 ml Diäthyläther gewaschen und in 25 ml Wasser gelöst. Die trübe Lösung wird eisgekühlt und mit Natriumhydrogencarbonat auf pH 7 gestellt. Der Niederschlag wird abfiltriert, mit Wasser gewaschen und Filtrat und Waschflüssigkeit auf eine Säule von 180 ml Amberlite XAD-2 gegeben. Die Säule wird mit Wasser und anschliessend mit Wasser:Isopropanol 85:15 durchgewaschen. Die das 7β-[2-(2-Amino-4-thiazolyl)-2-methoxyiminoacetamido]-3-cephem-4-carbonsäure-natriumsalz enthaltenden Fraktionen werden vereinigt und eingedampft. Das Natriumsalz wird mit Aceton verrieben, abfiltriert und getrocknet.

DS: Rf ~ 0,19 (n-Butanol:Eisessig:Wasser 67:10:23); UV-Spektrum (Äthanol): $\lambda_{max} = 233$ mμ ($\varepsilon = 15\,500$) und 290 mμ sh ($\varepsilon = 5500$).

Beispiel 2

a) Eine Lösung von 3,31 g 2-(2-tert.Butoxycarbonylamino-4-thiazolyl)-2-methoxyiminoessigsäure in 35 ml Tetrahydrofuran wird mit 1,50 g 1-Hydroxybenztriazol und 2,27 g N,N'-Dicyclohexylcarbodiimid versetzt und 2 Stunden bei 0° gerührt. Das Reaktionsgemisch wird mit einer Lösung von 3,66 g 7β-Amino-3-cephem-4-carbonsäure-diphenylmethylester in 45 ml Tetrahydrofuran versetzt und 1 Stunden bei 0° und 4 Stunden bei Raumtemperatur weitergerührt. Vom ausgefallenen Harnstoff wird abfiltriert, das Filtrat im Vakuum eingeengt und der Rückstand mit Essigsäureäthylester, Citratpuffer, gesättigter wässriger Natriumhydrogencarbonatlösung und gesättigter wässriger Natriumhydrogencarbonatlösung und gesättigter wässriger Natriumchloridlösung aufgearbeitet. Trocknen der organischen Phase über Natriumsulfat, Eindampfen im Vakuum, Säulenchromatographie des erhaltenen Rohproduktes an Kieselgel mit Toluol/Äthylacetat 4:1 als Elutionsmittel und Kristallisation des gereinigten Produktes aus Methylenchlorid/Diäthyläther ergibt den 7β-[2-(2-tert. Butoxycarbonylamino-4-thiazolyl)-2-methoxyiminoacetamido]-3-cephem-4-carbonsäure-diphenylmethylester vom Schmelzpunkt 154–157°; DS: Rf ~ 0,37 (Toluol/Äthylacetat 1:1); UV-Spektrum (in Äthanol): λmax = 234 mμ ($\varepsilon = 15\,500$), 295 mμ (Schulter); IR-Spektrum (in $CH_2Cl_2$): Absorptionsbanden bei 3400; 1792; 1775 sh; 1728; 1680; 1640; 1545 $cm^{-1}$.

Beispiel 3

Eine Lösung von 840 mg 2-(2-tert.Butoxycarbonylamino-4-thiazolyl)-2-methoxyiminoessigsäure in 9 ml Methylenchlorid und 0,26 ml Pyridin wird bei −15° unter Stickstoff mit 0,5 ml Diäthylphosphorobromidat versetzt und 30 Minuten bei dieser Temperatur gerührt. Nach Zugabe von 1,00 g 7β-Amino-3-cephem-4-carbonsäure-diphenylmethylester bei −15° wird die Reaktionslösung bei Raumtemperatur während 2 Stunden gerührt, dann mit Chloroform verdünnt, nacheinander mit verdünnter Schwefelsäure, Wasser, wässriger Natriumbicarbonatlösung und wässriger Natriumchloridlösung gewaschen, über Natriumsulfat getrocknet und im Vakuum eingedampft. Der Rückstand wird durch präparative Dickschichtchromatographie an Silicagel mit Toluol/Äthylacetat 1:1 gereinigt. Man erhält die im Beispiel 2 beschriebene Verbindung.

Beispiel 4

Eine Lösung von 15,0 g 7β-[2-(2-tert.Butoxycarbonylamino-4-thiazolyl)-2-methoxyiminoacet amido]-3-cephem-4-carbonsäure-diphenylmethylester in 75 ml Methylenchlorid und 75 ml Trifluoressigsäure wird 1 Stunde bei Raumtemperatur gerührt, mit kaltem Toluol versetzt und im Vakuum eingedampft. Der Rückstand wird mit Diäthyläther digeriert, abfiltriert und getrockent. Das erhaltene hellbeige, pulverförmige Trifluoracetat der 7β-[2-(2-Amino-4-thiazolyl)-2-methoxyiminoacetamido]-3-cephem-4-carbonsäure wird in 90 ml Wasser suspendiert. Der pH-Wert der Suspension wird mit 2N NaOH auf 7,1 gestellt. Die Lösung wird klarfiltriert und an 1200 ml Amberlite XAD-2 charomatographiert. Elution mit Wasser/15% Isopropanol und Lyophilisation der vereinigten, das gewünschte Produkt enthaltenden Fraktionen ergibt das schwach gelbliche 7β-[2-(2-Amino-4-thiazolyl)-2-methoxyiminoacetamido]-3-cephem-4-carbonsäure-natriumsalz.
IR-Spektrum (Nujol): Charakteristische Absorptionsbanden bei 3300 b; 1770; 1670 b; 1600; 1532; 1460 $cm^{-1}$.

Eine Lösung von 100 mg des erhaltenen Natriumsalzes in 5 ml Wasser werden mit 0,5N Salzsäure auf pH 3,5 eingestellt. Die ausgefallene 7β-[2-(2-Amino-4-thiazolyl)-2-methoxyiminoacetamido]-3-cephem-4-carbonsäure wird abfiltriert, mit Wasser und Aceton gründlich gewaschen und im Vakuum getrocknet.

Beispiel 5

Eine Lösung von 3,3 g 7β-[2-(2-tert.Butoxycarbonylamino-4-thiazolyl)-2-methoxyiminoacet amido]-3-cephem-4-carbonsäure-diphenylmethylester in 35 ml Methylenchlorid wird mit 15,4 ml Trifluoressigsäure 30 Min. bei 0° gerührt, mit kaltem Toluol versetzt und im Vakuum eingedampft. Der Rückstand wird mit Diäthyläther digeriert, filtriert und getrocknet. Das erhaltene gelbe Pulver wird in 10 ml Methanol gelöst, mit einer methanolischen Lösung von Natriumäthylhexanoat und, zur Vervollständigung der Fällung, mit Diäthyläther versetzt und 1,5 Std. bei Eiskühlung gerührt. Das erhaltene Natriumsalz der 7β-[2-(2-tert. Butoxycarbonylamino-4-thiazolyl)-2-methoxyimino-acetamido]-3-cephem-4-carbonsäure wird abfiltriert, mit Diäthyläther gut gewaschen und getrocknet. DS: Rf ~ 0,47 (n-Butanol:Essigsäure:Wasser 67:10:23); UV-Spektrum (Wasser): $\lambda max = 233$ mμ (ε = 15 500), 290 mμ (Schulter).

Zur Überführung in die freie Säure werden 1,5 g des erhaltenen Natriumsalzes in Äthylacetat suspendiert und mit 2N Salzsäure angesäuert. Die organische Phase wird mit Wasser und wässriger Natriumchloridlösung gewaschen, getrocknet und im Vakuum eingedampft. Der Rückstand wird an Silikagel (desaktiviert mit 5% Wasser) mit Methylenchlorid, Methylenchlorid/Äthylacetat (von 10 bis 50% steigend) und schliesslich reinem Äthylacetat chromatographiert. Die erhaltene 7β-[2-tert.Butoxycarbonylamino-4-thiazolyl)-2-methoxyiminoacetamido]-3-cephem-4-carbonsäure wird aus Methanol/Diäthyläther/Hexan umkristallisiert. Schmelzpunkt: um 210° (Zersetzung); DS: $Rf_{52A}$ ~ 0,42; UV-Spektrum (Äthanol): $\lambda max = 260$ nm (ε = 16 880); 232 nm (ε = 19 900); 224 nm (ε = 20 000); IR-Spektrum (Nujol); Absorptionsbanden bei 3270; 3180; 1782; 1718; 1654 $cm^{-1}$.

Beispiel 6

Eine Mischung von 2,25 ml Chlormethylpivalat und 9,0 g Natriumjodid in 30 ml Aceton wird 3 Std. bei Raumtemperatur gerührt. Das Gemisch wird mit einer Lösung von 2,6 g 7β-[2-(2-tert.Butoxycarbonylamino-4-thiazolyl)-2-methoxyiminoacetamido]-3-cephem-4-carbonsäure-natriumsalz in 50 ml Dimethylformamid versetzt und 1 Std. bei Raumtemperatur weitergerührt. Nach Einengen im Vakuum wird der Rückstand in Äthylacetat gelöst und die Lösung mit gesättigter wässriger Natriumchloridlösung gewaschen und über Natriumsulfat getrocknet. Das nach Eindampfen im Vakuum erhaltene Rohprodukt wird an Kieselgel chromatographiert. Elution mit Toluol enthaltend 20–30% Äthylacetat ergibt den 7β-[2-(2-tert.Butoxycarbonylamino-4-thiazolyl)-2-methoxyiminoacetamido]-3-cephem-4-carbonsäure-pivaloyloxymethylester. DS: Rf ~ 0,61 (Äthylacetat); UV-Spektrum (Äthanol): $\lambda max = 234$ mμ (ε = 1600), 293 mμ (Schulter); IR-Spektrum (in $CH_2Cl_2$): Absorptionsbanden bei 3380; 1789; 1773 sh; 1725; 1680; 1638; 1545 $cm^{-1}$.

Beispiel 7

Eine Lösung von 1,7 g 7β-[2-(2-tert.Butoxycarbonylamino-4-thiazolyl)-2-methoxyiminoacetamido]-3-cephem-4-carbonsäure-pivaloyloxymethylester in 8,7 ml Methylenchlorid wird mit 8,7 ml Trifluoressigsäure versetzt und 1 Std. bei Raumtemperatur gerührt. Nach Zugabe von kaltem Toluol wird im Vakuum eingedampft. Der Rückstand wird mit Diäthylätherhexan 1:1 verrührt, abfiltriert und getrocknet. Das erhaltene braune Pulver wird in Äthylacetat aufgenommen, mit kalter gesättigter Natriumhydrogencarbonat- und gesättigter wässriger Natriumchlorid-Lösung gewaschen, über Natriumsulfat getrocknet und im Vakuum eingedampft. Der Rückstand wird mit Diäthyläther digeriert, abfiltriert und im Hochvakuum getrocknet. Der erhaltene 7β-[2-(2-Amino-4-thiazolyl)-2-methoxyiminoacetamido]-3-cephem-4-carbonsäure-pivaloyloxymethylester zersetzt sich ab etwa 130 °C. DS Rf ~ 0,28 (Äthylacetat); UV-Spektrum (Äthylacetat): $\lambda max = 235$ mμ (ε = 16 320); 295 mμ (Schulter); IR-Spektrum (in $CH_2Cl_2$): Charakteristische Absorptionsbanden bei 3480; 3390; 3330; 1782; 1753; 1680; 1620; 1530 $cm^{-1}$.

Beispiel 8

Eine Mischung von 0,11 ml Chlormethylpivalat und 0,45 g Natriumjodid in 1,5 ml Aceton wird 3 Std. bei Raumtemperatur gerührt. Die Suspension wird anschliessend mit 0,202 g 7β-[2-(2-Amino-4-thiazolyl)-2-methoxyiminoacetamido]-3-cephem-4-carbonsäure-natriumsalz in 3 ml Dimethylformamid versetzt und 3 Std. bei Raumtemperatur weitergerührt. Das Reaktionsgemisch wird mit Äthylacetat verdünnt, mit gesättigter wässriger Natriumchlorid-Lösung ausgeschüttelt, über Natriumsulfat getrocknet und im Vakuum eingedampft. Der Rückstand wird an Silicagel mit Toluol-Äthylacetat 1:1 und Äthylacetat chromatographiert und ergibt den 7β-[2-(2-Amino-4-thiazolyl)-2-methoxyiminoacetamido]-3-cephem-4-carbonsäure-pivaloyloxymethylester.

**Patentansprüche**

1. 7β-Aminothiazolyl-acetamido-3-cephem-4-carbonsäure-verbindungen der Formel

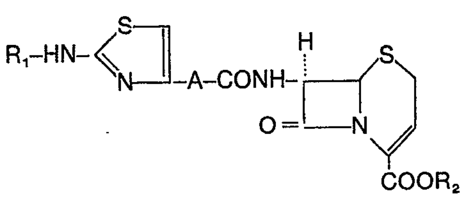

(I),

worin $R_1$ Wasserstoff oder eine Aminoschutzgruppe, A durch Methoxyimino substituiertes Methylen und $R_2$ Pivaloyloxymethyl darstellt, und Salze von solchen Verbindungen mit salzbildenden Gruppen.

2. 7β-[2-(2-tert.Butoxycarbonylamino-4-thiazolyl)-2-methoxyiminoacetamido]-3-cephem-4-carbonsäure-pivaloyloxymethylester, nach Patentanspruch 1.

3. 7β-[2-(2-Amino-4-thiazolyl)-2-methoxyiminoacetamido]-3-cephem-4-carbonsäure-pivaloyloxymethylester, nach Patentanspruch 1.

4. Pharmazeutische Präparate enthaltend eine der Verbindungen der Patentansprüche 1 oder 3.

5. Verbindungen nach einem der Patentansprüche 1 oder 3 zur Anwendung in einem Verfahren zur therapeutischen Behandlung des menschlichen oder tierischen Körpers.

6. Verbindungen nach einem der Patentansprüche 1 oder 3 zur Verwendung bei der Bekämpfung von bakteriellen Infektionen.

7. Verwendung von Verbindungen nach einem der Patentansprüche 1 oder 3 zur Herstellung von pharmazeutischen Präparaten.

8. Verfahren zur Herstellung von 7β-Aminothiazolylacetamido-3-cephem-4-carbonsäure-verbindungen der Formel I nach Patentanspruch 1 und Salzen von solchen Verbindungen mit salzbildenden Gruppen, dadurch gekennzeichnet, dass man zur Herstellung einer Verbindung der Formel I, worin $R_1$ Wasserstoff ist, und worin A und $R_2$ die unter Formel I gegebenen Bedeutungen haben, aus einer Verbindung der Formel I, worin $R_1$ eine Aminoschutzgruppe bedeutet, und A und $R_2$ die unter Formel I gegebenen Bedeutungen haben, die Aminoschutzgruppe $R_1$ abspaltet und durch Wasserstoff ersetzt, oder zur Herstellung einer Verbindung der Formel I, worin $R_2$ Pivaloyloxymethyl darstellt, und $R_1$ und A die unter Formel I genannten Bedeutungen haben, in einer Verbindung der Formel I, worin $R_2$ Wasserstoff ist, und worin $R_1$ und A die unter Formel I gegebenen Bedeutungen haben, die freie Carboxylgruppe in 4-Stellung des Cephemringes, oder ein reaktionsfähiges funktionelles Derivat davon, durch Behandlung mit einem den Pivaloyloxymethylrest einführenden Veresterungsmittel in eine veresterte Carboxylgruppe überführt.

9. Nach einem der Verfahren gemäss Patentanspruch 8 erhältliche Verbindungen.

**Claims**

1. A 7β-aminothiazolylacetamido-3-cephem-4-carboxylic acid compound of the formula

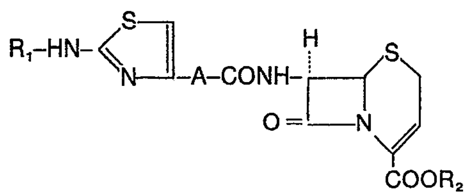

(I),

wherein $R_1$ is hydrogen or an amino protective group, A is methylene substituted by methoxyimino and $R_2$ is pivaloyloxymethyl, or a salt of such a compound containing a salt-forming group.

2. Pivaloyloxymethyl 7β-[2-(2-tert-butoxycarbonylamino-4-thiazolyl)-2-methoxyiminoacetamido]-3-cephem-4-carboxylate according to claim 1.

3. Pivaloyloxymethyl 7β-[2-(2-amino-4-thiazolyl)-2-methoxyiminoacetamido]-3-cephem-4-carboxylate according to claim 1.

4. A pharmaceutical preparation containing a compound as claimed in either of claims 1 or 3.

5. A compound as claimed in either of claims 1 or 3 for use in a therapeutic method of treating humans or animals.

6. A compound as claimed in either of claims 1 or 3 for use in the control of bacterial infections.

7. Use of a compound as claimed in either of claims 1 or 3 for the production of pharmaceutical preparations.

8. A process for the preparation of a 7β-aminothiazolylacetamido-3-cephem-4-carboxylic acid compound of the formula I as claimed in claim 1, or a salt of such a compound containing a salt-forming group, which process comprises splitting off the amino protective group from a compound of the formula I in which $R_1$ is an amino protective group and A and $R_2$ are as defined for formula I, and replacing it by hydrogen, to give a compound of the formula I in which $R_1$ is hydrogen and A and $R_2$ are as defined for formula I, or converting the free carboxyl group in the 4-position of the cephem ring, or a reactive functional derivative thereof, in a compound of the formula I in which $R_2$ is hydrogen and $R_1$ and A are as defined for formula I, by treatment with an esterifying agent which introduces the pivaloyloxymethyl radical, to give a compound of the formula I in which $R_2$ is pivaloyloxymethyl and $R_1$ and A are as defined for formula I.

9. A compound obtainable by a process as claimed in claim 8.

## Revendications

1. Dérivés de l'acide 7β-aminothiazolylacétamido-3-céphème-4-carboxylique de formule:

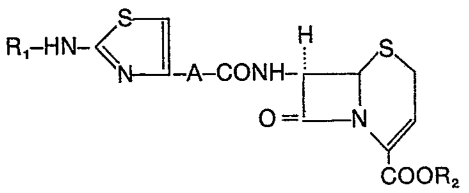

(I),

dans laquelle: $R_1$ représente l'hydrogène ou un groupe protecteur du groupe amino; A représente un groupe méthylène substitué par un groupe méthoxyimino et $R_2$ représente un groupe pivaloyloxyméthyle, et les sels de ces composés portant des groupes salifiables.

2. L'ester pivaloyloxyméthylique de l'acide 7β-[2-(2-tert.-butoxycarbonylamino-4-thiazolyl)-2-méthoxyimino-acétamido]-3-céphème-4-carboxylique selon la revendication 1.

3. L'ester pivaloyloxyméthylique de l'acide 7β-[2-(2-amino-4-thiazolyl)-2-méthoxyiminoacétamido]-3-céphème-4-carboxylique selon la revendication 1.

4. Compositions pharmaceutiques contenant l'un des composés des revendications 1 ou 3.

5. Composés selon l'une des revendications 1 ou 3, pour l'utilisation dans un procédé pour le traitement thérapeutique de l'organisme humain ou animal.

6. Composés selon l'une des revendications 1 ou 3, pour l'utilisation dans la lutte contre les infections bactériennes.

7. L'utilisation des composés selon l'une des revendications 1 ou 3 pour la préparation de compositions pharmaceutiques.

8. Procédé de préparation des dérivés de l'acide 7β-aminothiazolylacétamido-3-céphème-4-carboxylique de formule (I) selon la revendication 1 et des sels de ces composés portant des groupes salifiables, caractérisé en ce que: pour la préparation d'un composé de formule (I) dans laquelle $R_1$ représente l'hydrogène et A et $R_2$ ont les significations indiquées en référence à la formule (I), partant d'un composé de formule (I) dans laquelle $R_1$ représente un groupe protecteur du groupe amino, et A et $R_2$ ont les significations indiquées en référence à la formule (I), on élimine le groupe protecteur du groupe amino $R_1$ et on le remplace par l'hydrogène, ou bien pour la préparation d'un composé de formule (I) dans laquelle $R_2$ représente le groupe pivaloyloxyméthyle, et $R_1$ et A ont les significations indiquées en référence à la formule (I), partant d'un composé de formule (I) dans laquelle $R_2$ représente l'hydrogène et $R_1$ et A ont les significations indiquées en référence à la formule (I), on convertit le groupe carboxyle libre en position 4 du cycle céphème, ou un dérivé fonctionnel réactif de ce groupe, par traitement à l'aide d'un agent estérifiant introduisant le reste pivaloyloxyméthyle, en un groupe carboxyle estérifié.

9. Composés susceptibles d'être obtenus par l'un des procédés de la revendication 8.